Europäisches Patentamt

European Patent Office

Office européen des brevets

(11) Veröffentlichungsnummer: **0 022 296**
**B1**

(12) # EUROPÄISCHE PATENTSCHRIFT

(45) Veröffentlichungstag der Patentschrift:
**01.12.82**

(51) Int. Cl.³: **A 61 B 6/02**

(21) Anmeldenummer: **80200626.2**

(22) Anmeldetag: **01.07.80**

(54) Röntgenuntersuchungsgerät für Schicht- und andere Aufnahmeverfahren.

(30) Priorität: **06.07.79 DE 2927380**

(43) Veröffentlichungstag der Anmeldung:
**14.01.81 Patentblatt 81/2**

(45) Bekanntmachung des Hinweises auf die Patenterteilung:
**01.12.82 Patentblatt 82/48**

(84) Benannte Vertragsstaaten:
**DE FR GB IT NL SE**

(56) Entgegenhaltungen:
**DD-A-121 600**
**DE-B-2 119 864**
**US-A-3 838 286**

(73) Patentinhaber: **Philips Patentverwaltung GmbH,
Steindamm 94, D-2000 Hamburg 1 (DE)**
(84) Benannte Vertragsstaaten: **DE**

(73) Patentinhaber: **N.V. Philips' Gloeilampenfabrieken,
Groenewoudseweg 1, NL-5621 BA Eindhoven (NL)**
(84) Benannte Vertragsstaaten: **FR GB IT NL SE**

(72) Erfinder: **Onken, Volker, Alsterkrugchaussee 312,
D-2000 Hamburg 60 (DE)**

(74) Vertreter: **Hartmann, Heinrich, Dipl.-Ing. et al, Philips
Patentverwaltung GmbH Steindamm 94,
D-2000 Hamburg 1 (DE)**

## Röntgenuntersuchungsgerät für Schicht- und andere Aufnahmeverfahren

Die Erfindung bezieht sich auf ein Röntgenuntersuchungsgerät für Schicht- und andere Aufnahmeverfahren mit einem an einem horizontalen Röhrentragarm befestigten bei Schichtaufnahmebetrieb in einer horizontalen Ebene oberhalb einer Patientenlagerungsvorrichtung bewegbaren Röntgenstrahler und einer unterhalb der Patientenlagerungsvorrichtung in einer horizontalen Ebene bewegbaren Bildaufnahmeeinrichtung, wobei zur gegensinnigen Verschiebung von Röntgenstrahler und Bildaufnahmeeinrichtung eine Kupplungsvorrichtung vorgesehen ist, die aus zwei teleskopartig gegeneinander verschiebbaren, um eine horizontale Achse schwenkbaren Teilen besteht, von denen der eine mit der Bildaufnahmeeinrichtung und der andere lösbar mit dem Röhrentragarm verbunden ist.

Ein derartiges Gerät ist unter der Bezeichnung «Schichteinrichtung LT» der Firma Philips bekannt. Die Kupplungsvorrichtung besteht dabei aus einer Hülse, die mit der Bildaufnahmevorrichtung, einer Laufrastereinrichtung für Filmaufnahmen, gelenkig, im übrigen aber fest verbunden ist, und einer in der Hülse gleitenden Schichtstange, die lösbar mit dem Röhrentragarm verbunden ist. Die Hülse ist in einer Muffe geführt, die um eine horizontale, senkrecht zur Längsrichtung des Patientenlagerungstisches verlaufende Achse drehbar ist, die die scharf abgebildete Schichtebene bestimmt.

Wenn mit einem solchen Röntgenuntersuchungsgerät keine Schichtaufnahmen durchgeführt werden sollen, sondern andere Aufnahmen, z.B. eine Wandstativ-Aufnahme, dann muss die Kupplungsvorrichtung vom Röhrentragarm entkuppelt werden. Zu diesem Zweck muss der Benützer um das Gerät herum auf die Seite gehen, auf der sich die Kupplungsvorrichtung befindet, und eine an der Schichtstange angebrachte Klinke lösen und die Schichtstange von dem Röhrentragarm trennen. Er benötigt dazu beide Hände.

Weiterhin ist aus der US-A 3 838 286 ein Röntgenuntersuchungsgerät bekannt, mit dem Schichtaufnahmen, Schrägaufnahmen und Bukky-Aufnahmen gemacht werden können. Die Kupplungsvorrichtung besteht dabei aus einer einzigen Stange, die bei Schrägaufnahmen und bei Schichtaufnahmen mit dem Tragarm gekuppelt bleibt, wobei im letzten Fall die Stange gleitend in dem Röhrentragarm geführt ist, während er im ersten Fall fest mit der Stange verbunden ist. Zu diesem Zweck ist die Schichtstange mit Rippen versehen, in die am Tragarm befestigte Klemmbacken eingreifen. Wenn die Schichtstange vom Röhrentragarm entkoppelt werden soll, muss der Benützer ebenfalls um das Gerät herum gehen, zwei am unteren und oberen Ende der Schichtstange angebrachte Rastmechanismen betätigen und die Stange aus ihrer für Schicht- oder Schrägaufnahmen erforderlichen Position in eine Parkposition heben.

Aufgabe der vorliegenden Erfindung ist es, ein Röntgenuntersuchungsgerät der eingangs genannten Art so auszubilden, dass das Abkoppeln der Kupplungsvorrichtung vom Röhrentragarm wesentlich vereinfacht ist.

Diese Aufgabe wird erfindungsgemäss dadurch gelöst, dass der mit dem Röhrentragarm verbundene Teil der Kupplungsvorrichtung an seinem oberen Ende mit einer Querschnittsveränderung versehen ist, dass am Röhrentragarm eine Rastvorrichtung befestigt ist, die in die Querschnittsveränderung eingreift, dass ein Anschlag vorgesehen ist, der beim Herunterfahren des Röhrentragarms den Teil relativ zum Röhrentragarm nach oben drückt, dass die Rastvorrichtung und die Querschnittsveränderung so ausgebildet sind, dass nur bei einer Aufwärtsbewegung des mit dem Röhrentragarm verbundenen Teils der Kupplungsvorrichtung die Rastvorrichtung aus der Raststellung ausrückbar ist, und dass ein Arretiermechanismus bei Betätigung durch den Benützer die Rastvorrichtung in der ausgerückten Position festhält.

Durch das Einrasten der Rastvorrichtung in die Querschnittsveränderung an dem mit dem Röhrentragarm verbundenen Teil werden dieser Teil und der Röhrentragarm (lösbar) miteinander verbunden. Das Abkoppeln dieser Kupplungsvorrichtung erfolgt dabei wie folgt:

Der Benutzer zieht den Röhrentragarm nach unten, indem er z.B. den an dem Röhrentragarm befestigten Röntgenstrahler mittels daran angebrachter Handgriffe nach unten bewegt. Dabei wird auch der mit dem Röhrentragarm verbundene Teil der Kupplungsvorrichtung nach unten bewegt, bis dieser auf den Anschlag stösst. Dadurch wird er (relativ zum Röhrentragarm) nach oben gedrückt, wodurch die Rastvorrichtung ausrücken kann. Wenn jetzt der Arretiermechanismus betätigt ist, wird die Rastvorrichtung in der ausgerückten Stellung festgehalten, so dass der Röhrentragarm nach oben bewegt werden kann (der mit dem Röhrentragarm verbindbare Teil verschiebt sich dann in bezug auf den Röhrentragarm nach unten), ohne dass die Rastvorrichtung einrastet. Die Kupplungsvorrichtung ist dann entkoppelt und der mit dem Röhrentragarm verbindbare Teil ist auf dem Anschlag abgestellt. – Zum Ankoppeln wird der Röhrentragarm nach unten gefahren, wobei die Rastvorrichtung selbsttätig einrastet – falls die Arretiervorrichtung nicht betätigt ist.

Eine Weiterbildung der Erfindung sieht vor, dass die Querschnittsveränderung eine Nut ist. Grundsätzlich könnte die Querschnittsveränderung aber auch durch eine Verdickung gebildet werden, z.B. durch einen Flansch an dem mit dem Röhrentragarm verbindbaren stangenartigen Teil.

Eine Weiterbildung der Erfindung sieht vor, dass die Rastvorrichtung einen an einem Ende angelenkten Hebel umfasst, der mit einem in die Nut passenden Sperrstift versehen ist, dass der mit dem Röhrentragarm verbindbare Teil der Kupplungsvorrichtung zwischen dem Gelenkpunkt des Hebels und dem Stift geführt ist, und dass der Hebel mit Federkraft nach unten gedrückt wird. Eine solche Rastvorrichtung ist verhältnismässig einfach.

Eine andere Weiterbildung der Erfindung sieht vor, dass in dem Röhrentragarm eine Hülse mit sich nach oben konisch erweiternder Bohrung befestigt ist, in die das obere, sich nach oben konusförmig verbreiternde Ende des mit dem Röhrentragarm verbindbaren Teils der Kupplungsvorrichtung einführbar ist, wobei die Durchmesserzunahme bei der Hülse grösser ist als bei dem Teil, und dass in der Hülse wenigstens zwei auf dem Umfang mit Abstand zueinander sitzende, sich nach oben ebenfalls konisch erweiternde Klemmbackenteile vorgesehen sind, die das obere Ende des Teils aufnehmen und bei einer Abwärtsbewegung in der Hülse festhalten, wobei der Arretiermechanismus die Klemmbacken in einer oberen Position festhält. Diese Ausgestaltung hat den Vorteil, dass der mit dem Röhrentragarm verbundene Teil der Kupplungsvorrichtung, im allgemeinen eine Stange mit kreisförmigem Querschnitt, in der festgeklemmten (Rast-)Stellung eine definierte Position in bezug auf die Hülse und damit auch auf den Tragarm einnimmt, ohne jegliches Spiel. Dadurch ist eine exakte Ausrichtung von Röntgenstrahler und Bildaufnahmeeinrichtung gewährleistet, was der Aufnahmequalität zugute kommt.

Eine andere Weiterbildung der Erfindung sieht vor, dass im Röhrentragarm eine Feder angeordnet ist, die den mit dem Röhrentragarm rastend verbundenen Teil der Kupplungsvorrichtung nach unten drückt. Diese Feder verhindert einerseits, dass beim Herunterfahren des Röhrentragarms das obere Ende des mit diesem verbundenen Teils der Kupplungsvorrichtung gegen den Röhrentragarm fährt und wirkt als Stossdämpfer; ausserdem drückt sie diesen Teil mit einer definierten Kraft in die Raststellung, so dass dieser Teil der Kupplungsvorrichtung sich auch bei Erschütterungen des Gerätes nicht versehentlich aus der Raststellung lösen kann.

Die Erfindung wird nachstehend anhand eines in der Zeichnung dargestellten Ausführungsbeispiels näher erläutert. Es zeigen

Fig. 1 eine Gesamtansicht des erfindungsgemässen Röntgenuntersuchungsgerätes,

Fig. 2 eine schematische Schnittzeichnung eines Teils des Röhrentragarms und der Kupplungsvorrichtung im eingerasteten Zustand,

Fig. 3 die gleiche Ansicht, jedoch im ausgerasteten Zustand,

Fig. 4 und 5 ein anderes Ausführungsbeispiel im seitlichen Schnitt und in der Draufsicht,

Fig. 6 bis 7 andere Ausführungsbeispiele der Erfindung.

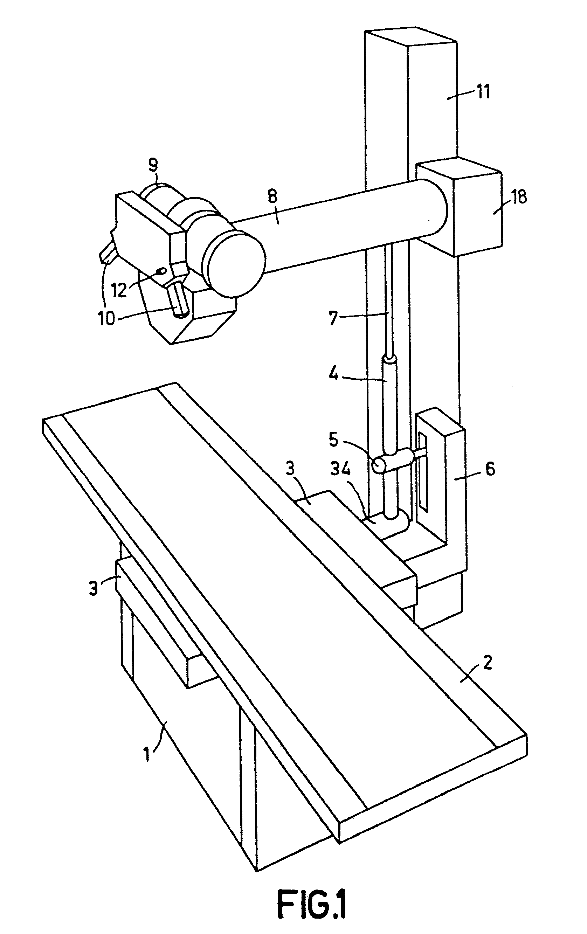

Das in Fig. 1 dargestellte Röntgenuntersuchungsgerät umfasst eine Patientenlagerungsvorrichtung, die aus einem Gerätefuss 1 und einer darauf befestigten horizontalen angeordneten Patientenlagerungsplatte 2 besteht. Unter der Patientenlagerungsplatte ist eine Bildaufnahmeeinrichtung 3 in einer horizontalen Ebene bewegbar angeordnet. Die Bildaufnahmeeinrichtung kann z.B. durch eine Laufrastereinrichtung gebildet werden, die ein Raster und eine damit verbundene Ladeeinrichtung zur Aufnahme einer mit einem Film versehenen Kassette umfasst.

An die Bildaufnahmeeinrichtung 3 ist bei 34 das untere Ende einer hülsenförmigen Stange 4, d.h. einer Stange mit kreisringförmigem Querschnitt, angelenkt. Die Stange 4 ist in einer Muffe 5 geführt, die in einem Schichtbock 6 um eine horizontale, zur Tischlängsrichtung senkrechte Achse drehbar gelagert ist. Durch Verstellen der Muffe in vertikale Richtung innerhalb des Schichtbockes 6 wird die Lage der bei der Aufnahme scharf abgebildeten Schicht festgelegt. Eine in der Hülse 4 teleskopartig verschiebbar angeordnete Schichtstange 7 ist mit dem Röhrentragarm 8 verbunden. Sie bildet zusammen mit der Hülse 4 die Kupplungsvorrichtung zwischen der Bildaufnahmeeinrichtung 3 und dem Röhrentragarm 8 bzw. dem daran befestigten Röntgenstrahler 9. Der sich horizontal und senkrecht zur Längsrichtung des Patientenlagerungstisches 2 erstreckende Röhrentragarm 8 ist um seine Längsachse drehbar an einem Halteteil 18 befestigt, der seinerseits in vertikaler Richtung verstellbar an einer vertikalen Stativsäule 11 befestigt ist, die in Längsrichtung des Tisches verfahrbar ist.

In der Position der Stativsäule 11, in der Röntgenstrahler 9 und Bildaufnahmeeinrichtung 3 senkrecht übereinander stehen, ist eine Raststellung vorgesehen. In dieser Stellung erfolgt auch das Abkuppeln der Schichtstange 7 vom Röhrentragarm 8.

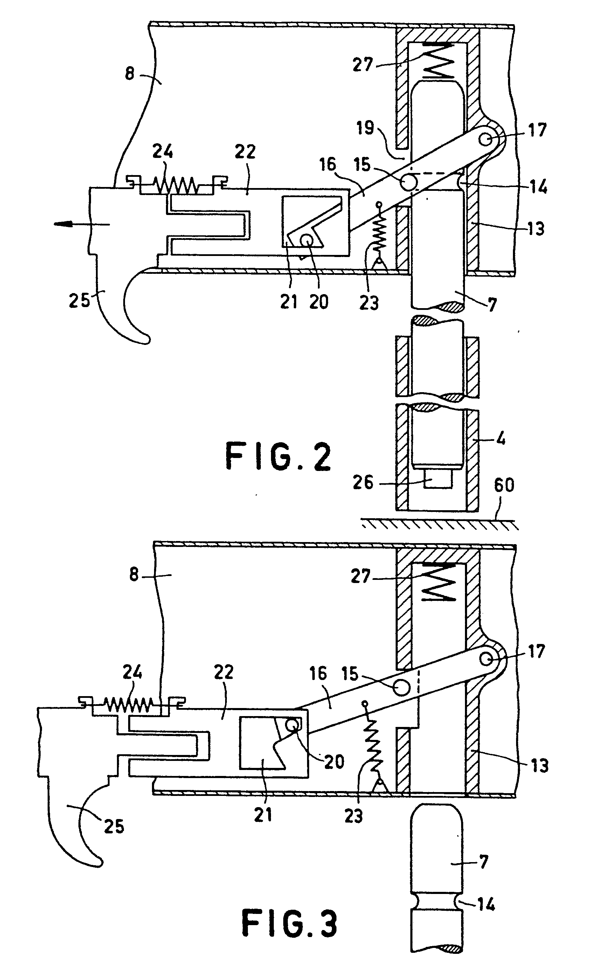

Wie noch näher in Verbindung mit Fig. 2 und 3 erläutert, erfolgt das Abkuppeln dadurch, dass der Benutzer den Röhrentragarm 8 bzw. den daran befestigten Röntgenstrahler 9 mittels der Handgriffe 10 nach unten fährt, bis die Schichtstange 7, die länger ist als die Hülse 4, mit ihrem unteren Ende gegen einen Anschlag stösst, der durch eine horizontale Fläche des Schichtbockes 6 gebildet werden kann. Wenn der Benutzer dabei zugleich mit dem am Röntgenstrahler 9 angebrachten Knopf 12 den Arretiermechanismus betätigt, wird die Schichtstange 7 entkoppelt, wenn der Röntgenstrahler 9 wieder nach oben geschoben wird. Das Ankoppeln, d.h. die Verbindung der Schichtstange 7 mit dem Röhrentragarm 8 erfolgt dann dadurch, dass das Stativ 11 in die Raststellung geschoben wird, wonach der Röntgenstrahler abgesenkt wird und die Rastvorrichtung einrastet.

Wie Fig. 2 zu entnehmen ist, ist im Inneren des Röhrentragarms 8, der im wesentlichen aus einem zylinderförmigen Hohlprofil besteht, senkrecht zur Längsrichtung des Röhrentragarms 8 eine Passhülse 13 zur Aufnahme der Schichtstange 7 vorgesehen. Die Schichtstange 7 ist in der Nähe ihres oberen Endes mit einer Querschnittsveränderung in Form einer Nut 14 versehen, in die ein Sperrstift 15 durch eine Öffnung 19 in der Passhülse 13 einrastet. Der Sperrstift 15 ist an einem Hebel 16 befestigt, der an seinem einen Ende, das sich auf der anderen Seite der Schichtstange 7 befindet, an der Passhülse 13 bzw. dem Röhrentragarm 8 bei 17 angelenkt ist und der an seinem anderen Ende mit einem Stift 20 versehen ist, der in eine Ausnehmung 21 einer Steuerkulisse 22 eingreift. Das letztgenannte Ende wird durch eine am Röhrentragarm 8 befestigte Feder 23 nach unten gezogen. Die Steuerkulisse 22 ist über eine Feder 24 mit einem nur teilweise dargestellten Hebelgestänge 25 verbunden, das bei Betätigen des am Röntgenstrahler angebrachten Knopfes 12 (Fig. 1) in Pfeilrichtung verschoben wird.

Am unteren Ende der in der Hülse 4 gleitenden Schichtstange 7 befindet sich ein Gummipuffer 26 und unterhalb der Hülse 4 befindet sich eine als Anschlag 60 dienende horizontale Fläche. – Eine Feder 27 wird in der in Fig. 2 dargestellten Raststellung der Schichtstange zusammengedrückt; sie übt daher eine nach unten gerichtete Kraft auf die Schichtstange aus. Zum Abkoppeln der Schichtstange 7 betätigt der Benutzer am Knopf 12 das Hebelgestänge 25, wodurch sich diese in Pfeilrichtung verschiebt. Dadurch wird die Feder 24 gespannt und der Stift 20 verschiebt sich in dem Ausschnitt 21 der Steuerkulisse 22 ganz nach rechts. Fährt der Benutzer nun den Röhrentragarm an den Handgriffen 10 (Fig. 1) nach unten, bis der Gummipuffer 26 am unteren Ende der Schichtstange 7 auf den Anschlag 60 trifft, dann wird die Schichtstange 7 in der Passhülse 13 entgegen der Kraft der Feder 27 nach oben verschoben. Dabei wird das freie linke Ende des Hebels 16 mit nach oben geschwenkt bis der Sperrstift 15 ganz aus der Nut 14 herausgetreten ist. Durch diese Bewegung verschiebt sich der Stift 20 in der Ausnehmung 21 der Steuerkulisse 22 hoch und wird, da die Steuerkulisse 22 durch die Feder 24 in Pfeilrichtung gezogen wird, in der rechten oberen Position festgehalten (Fig. 3). Wird in dieser Situation der Röhrentragarm 8 bzw. der Röntgenstrahler 9 nach oben gefahren, bewegt sich die Schichtstange 7 in bezug auf den Röhrentragarm 8 bzw. die Passhülse 13 nach unten, wobei die Rastvorrichtung, d.h. der Sperrstift 15, nicht in die Nut 14 einrasten kann, weil der Hebel 16 über den Stift 20 in seiner oberen, ausgerückten Position arretiert wird.

Soll die Schichtstange wieder mit dem Röhrentragarm 8 verbunden werden, wird der Röhrentragarm nach unten bewegt, wobei sich die Passhülse 13 über das obere Ende der Schichtstange 7 schiebt, bis der Stift 15 von der Schichtstange 7 erfasst wird. Die Schichtstange drückt den Stift 15 nach aussen und gleitet in der Passhülse 13 weiter, bis die Nut 14 sich auf Höhe des Sperrstiftes 15 befindet. Dieser rastet in die Nut 14 ein, weil die Steuerkulisse 22 dann in der in Fig. 2 dargestellten Position, d.h. der Knopf 12 und das Hebelgestänge 25 sind nicht betätigt.

Das Einführen der Schichtstange 7 in die Passhülse 13 beim Einkuppeln wird erleichtert, wenn die obere Stirnfläche der Schichtstange 7 an den Rändern leicht abgerundet ist, wie in Fig. 2 und 3 dargestellt. – Der aus dem Röhrentragarm 8 herausragende Griff des Hebelgestänges 25 gestattet es dem Benutzer, das Abkoppeln der Schichtstange 7 notfalls auch von der Seite der Patientenlagerungsvorrichtung 1, 2 aus vorzunehmen, an der sich das Stativ 11 (Fig. 1) befindet.

Mit Vorteil wird anstelle eines mechanischen Hebelgestänges zum Verschieben der als Arretiermechanismus dienenden Steuerkulisse 22 ein elektrischer Hubmagnet verwendet, der durch Betätigen des Knopfes 12 erregt wird und dabei die Steuerkulisse 22 nach links zieht, die durch eine Rückholfeder bei nicht erregtem Hubmagneten wieder nach rechts in ihre Ausgangsposition (Fig. 2) gezogen werden kann.

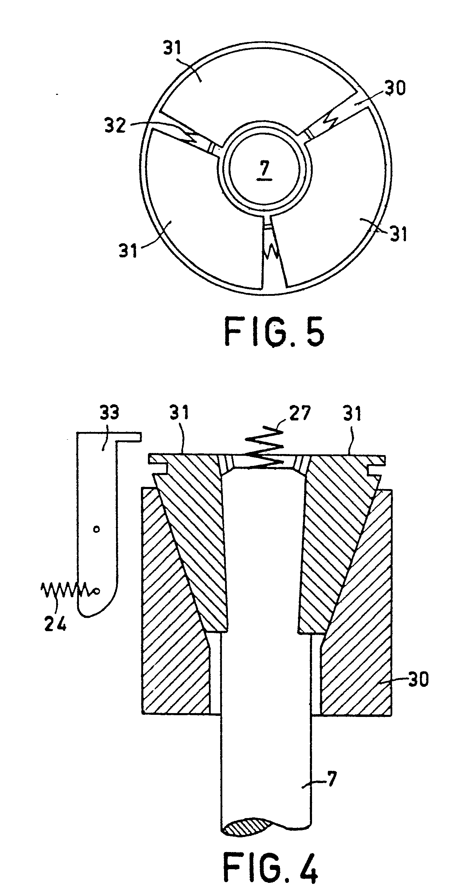

Bei der in den Fig. 2 und 3 dargestellten Ausführungsform besitzt die Schichtstange 7 innerhalb der Passhülse 13 ein gewisses seitliches Spiel (d.h. ihr Aussendurchmesser ist etwas geringer als der Innendurchmesser der Passhülse), damit sie innerhalb der Passhülse gleiten kann. Dieses Spiel bleibt aber auch im eingerasteten Zustand erhalten. Das kann dazu führen, dass die Verbindungslinie zwischen dem Brennfleck des Röntgenstrahlers und dem Mittelpunkt der Bildaufnahmevorrichtung 3 sich entsprechend diesem Spiel verschiebt, was gewisse Unschärfen zur Folge haben kann. In den Fig. 4 und 5 ist eine Ausführungsform beschrieben, bei der im festgeklemmten Zustand überhaupt kein Spiel auftritt.

Fig. 4 zeigt eine auf nicht näher dargestellte Weise mit dem Röhrentragarm verbundene zylinderförmige Hülse 30. Die zylinderförmige Hülse 30 besitzt eine Bohrung, die sich nach oben hin konisch erweitert (Fig. 4). Die Bohrung in der Hülse 30 ist so gross, dass das obere Ende der Schichtstange 7 ohne weiteres hindurchpasst. Die Schichtstange 7 weist an ihrem oberen Ende eine Einschnürung auf, so dass eine Schulter gebildet wird, oberhalb der der Durchmesser der Schichtstange 7 allmählich wieder zunimmt. Das oberhalb der erwähnten Schulter gelegene Ende der Schichtstange 7 ist somit auch konisch, jedoch weniger stark als die Hülse 30, d.h. der Winkel, den das obere Ende der Schichtstange 7 mit der Vertikalen bildet, ist kleiner als bei der Hülse 30. Die Rastvorrichtung wird in diesem Fall durch drei auf dem Umfang mit gleichmässigem Abstand zueinander sitzende, sich nach oben konusförmig erweiternde Klemmbacken 31 gebildet. Der Winkel zwischen den äusseren und inneren Mantellinien dieser konusförmigen Klemmbacken 31 entspricht dem Winkel zwischen der Mantellinie der Innenfläche der Hülse und der des oberen Endes der Schichtstange. Zwischen den Klemmbacken 31 befinden sich Federn 32, die die Klemmbacken auseinanderdrücken. Es kann auch noch eine Feder vorgesehen sein, die auf die oberen Stirnflächen der Klemmbacken 31 eine nach unten gerichtete Kraft ausübt.

Wie aus Fig. 4 ersichtlich, wird das obere Ende der Schichtstange 7 durch die Klemmbacken 31 festgeklemmt, wenn auf die Schichtstange 7 eine nach unten gerichtete Kraft (z.B. ihr Gewicht in Kombination mit der Kraft der Druckfeder 27) wirkt. Die Klemmbacken haben hier also eine ähnliche Funktion wie die Klemmbacken bei dem Bohrfutter einer Bohrmaschine. Statt dreier Klemmbacken können auch zwei, jedoch auch vier oder mehr vorgesehen sein. Wenn die Schichtstange auf diese Weise festgeklemmt ist, ist kein seitliches Spiel mehr vorhanden.

Auch hier wird die Rastvorrichtung dadurch gelöst bzw. ausgerückt, dass der Röntgenstrahler 9 zusammen mit dem die Hülse 30 enthaltenden Röhrentragarm 8 nach unten gefahren wird, bis das untere Ende der Schichtstange 7 gegen einen Anschlag stösst. Dadurch wird eine aufwärts gerichtete Kraft auf die Schichtstange 7 erzeugt, die die Klemmbacken 31 nach oben aus der Hülse 30 herausschiebt, wodurch sich der Innendurchmesser der Klemmbackenanordnung vergrössert. Werden dann die Klemmbacken z.B. durch einen von dem Benutzer über ein nicht näher dargestelltes Hebelgestänge und die Feder 24 zu betätigende Arretierhebel 33 in ihrer oberen Position festgehalten, dann kann die Schichtstange 7 leicht entkoppelt werden, indem der Röhrentragarm nach oben verschoben wird, wobei das obere Ende der Schichtstange 7 nach unten aus der Klemmbackenanordnung 31, deren Innendurchmesser dann vergrössert ist, herausgefahren wird.

Der Einfachheit halber ist in Fig. 4 nur ein einziger Arretierhebel 33 gezeichnet, während in Fig. 5 die Arretierhebel ganz weggelassen sind. Grundsätzlich benötigt aber jede Klemmbacke 31 einen Arretierhebel 33. – Anstelle eines Arretierhebels könnte auch ein ringförmiger, über der oberen Stirnfläche der Klemmbacken 31 angeordneter Elektromagnet vorgesehen sein, der die Klemmbacken 31 in einer oberen Position festhält, wenn er erregt ist, wobei die Erregung des Elektromagneten durch den Benutzer einzuschalten wäre. Durch eine innerhalb oder ausserhalb des ringförmigen Elektromagneten angeordnete Ringfeder, die ebenfalls auf die obere Stirnfläche der Klemmbacken 31 wirkt, könnten die Klemmbacken 31 dabei nach unten gedrückt werden, wenn der erwähnte Elektromagnet nicht erregt ist.

Das Einkuppeln kann bei der in Fig. 4 und 5 dargestellten Anordnung auf ähnliche Weise erfolgen wie bei der in Fig. 2 und 3 dargestellten Anordnung:

Der Röhrentragarm wird nach unten gefahren, wobei das obere Ende der Schichtstange 7 die Klemmbacken 31 soweit nach oben drückt, bis der Innendurchmesser der aus den Klemmbacken 31 gebildeten Anordnung soweit zugenommen hat, dass die Schichtstange hindurchpasst und die unteren Stirnseiten der Klemmbacken 31 auf den durch die Einschnürung gebildeten Schultern in der Schichtstange aufsitzen. Wird dann der Röhrentragarm nach oben bewegt, dann erzeugen die Druckfeder 27 und das Gewicht der Schichtstange 7 eine nach unten gerichtete Kraft, wodurch die Schichtstange 7 festgeklemmt wird.

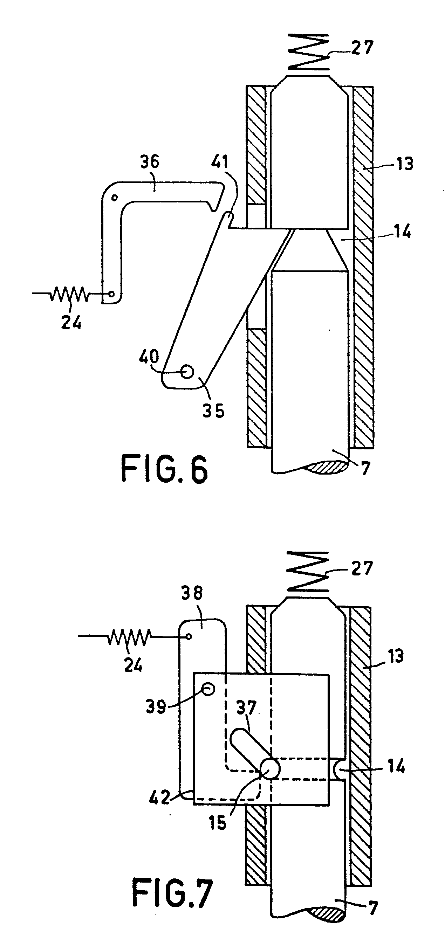

Bei dem in Fig. 6 dargestellten Ausführungsbeispiel sind ebenso wie in den Fig. 4 und 5 der Röhrentragarm und das untere Ende der Schichtstange 7 nicht dargestellt, sie sind aber genauso angeordnet wie bei den Fig. 1 bis 3. Auch hier ist mit dem Röhrentragarm eine Passhülse 13 fest verbunden, in der mit geringem seitlichen Spiel die Schichtstange 7 gleiten kann. Die Querschnittsveränderung 14 am oberen Ende der Schichtstange 7 besteht darin, dass in gewissem Abstand von der oberen Stirnfläche der Schichtstange 7 der Durchmesser der Schichtstange konusförmig nach oben hin abnimmt, wobei dieser konusförmige Teil der Schichtstange durch eine senkrecht zur Längsrichtung der Schichtstange verlaufende Schulter abgeschlossen wird, oberhalb der die Schichtstange wieder den normalen Durchmesser hat. In die so gebildete Querschnittsveränderung greift ein Hebel 35 ein, der um eine Achse 40 schwenkbar mit dem Röhrentragarm fest verbunden und so ausgebildet ist, dass er genau in die konische Ausnehmung 14 der Schichtstange 7 passt.

Wenn auf die Schichtstange 7 eine nach unten gerichtete Kraft (die Schwerkraft und die Kraft der Druckfeder 27) wirkt, trägt der Hebel 35 die Schichtstange 7, wobei dieser eine definierte Lage zur Passhülse 13 und damit zum Röhrentragarm einnimmt. Wenn nun der Röhrentragarm nach unten bewegt wird, gleitet die Schichtstange 7 in der Passhülse 13 nach oben, wobei der Hebel 35, der als Rastvorrichtung wirkt, nach aussen geschoben wird. Der Hebel 35 ist mit einer Nase 41 versehen, hinter die ein geeignet ausgebildeter Hebel 36 einrastet, wenn der Benutzer ein nicht näher dargestelltes Hebelgestänge betätigt, das die Feder 24 nach links zieht, oder einen Hubmagneten, der auf den Hebel 36 in der gleichen Richtung eine Kraft ausübt. Dabei wird das rechte Ende des Hebels 36 nach unten gekippt und greift hinter der Nase 41 ein und hält diese fest, so dass die Schichtstange 7 nach unten aus der Passhülse 13 herausbewegt werden kann (durch Verschieben des Röhrentragarms nach oben). Das Ankoppeln der Schichtstange 7 erfolgt, wenn die Arretiervorrichtung nicht mehr betätigt ist (der Arretierhebel 36 nimmt dann seine in Fig. 6 dargestellte Position ein), wobei der Hebel 35 mit seinem oberen Ende nach rechts fällt. Wird dann der Röhrentragarm nach unten geschoben, schiebt sich die Schichtstange 7 in der Passhülse 13 hoch, wobei der Hebel 35 in die Querschnittsveränderung 14 fällt, so dass die Schichtstange 7, wenn der Röhrentragarm wieder nach oben bewegt wird, durch den Hebel 35 in einer definierten Position bezüglich der Passhülse 13 eingerastet ist.

Eine weitere Ausführungsform ist in Fig. 7 dargestellt. Die Rastvorrichtung wird dabei wiederum durch einen Stift 15 gebildet, der in eine entsprechend geformte Nut 14 eingreift. Der Stift 15 ist in einem Langloch 37 verschiebbar, das in einer mit dem Röhrentragarm 8 auf nicht näher dargestellte Weise verbundenen Platte 42 angebracht ist. Das Langloch 37 verläuft schräg nach oben bzw. aussen (in bezug auf die Passhülse 13). Wird nun beim Herunterfahren des Röhrentragarms die Schichtstange 7 in der Passhülse 13 nach oben bewegt, wird der Stift 15 nach oben bzw. nach aussen mitgenommen, bis er aus der Nut 14 ausgetreten ist. Wenn der Benutzer dann über ein Hebelgestänge oder einen Elektromagneten den um den Punkt 39 an der Platte 42 schwenkbar befestigten Arretierhebel 38 betätigt, wird dessen unteres rechtes Ende zur Schichtstange 7 hin bzw. weiter in die Passhülse 13 hinein verschoben, so dass der Stift 15 in einer oberen Position festgehalten wird, in der er nicht mehr in die Nut 14 einrasten kann, wenn die Schichtstange 7 nach unten bewegt wird (durch Hochfahren des Röhrentragarms 8). Das Ankoppeln kann wieder erfolgen, wenn die Arretiervorrichtung vom Benutzer nicht betätigt ist, und der Röhrentragarm nach unten über die Schichtstange 7 gefahren ist, wobei der Sperrstift 15 in die Nut 14 einrastet. Dabei ist es von Vorteil, wenn z.B. durch eine nicht näher dargestellte Druckfeder auf den Stift 15 in Richtung des Langloches 37 eine nach unten und zur Schichtstange 7 gerichtete Kraft wirkt.

Allen Ausführungsbeispielen ist gemeinsam, dass die Schichtstange 7 nur dann vom Röhrentragarm entkoppelt werden kann, wenn dieser nach unten gefahren ist (in eine Position, die unter den bei Schichtaufnahmen üblichen Positionen des Röhrentragarms liegt) und wenn die die Rastvorrichtung in ihrer ausgerückten Position festhaltende Arretiervorrichtung (am Knopf 12, Fig. 1) betätigt ist. Dies stellt einerseits sicher, dass die Schichtstange nicht ungewollt entkoppelt werden kann, wenn nur der Betätigungsknopf 12 vom Benutzer betätigt ist (und der Röhrentragarm nicht nach unten gefahren ist), und andererseits wird die entkoppelte Schichtstange 7 gleich in dieser Entkopplungsposition auf dem Anschlag 60 abgestellt. Das Herunterfahren des Röhrentragarms zum Entkoppeln oder zum Ankoppeln sowie das Betätigen des Arretiermechanismus kann der Benutzer durchführen, ohne dass er auf die Seite des Gerätes gehen muss, auf der sich die Schichtstange befindet.

**Patentansprüche**

1. Röntgenuntersuchungsgerät für Schicht- und andere Aufnahmeverfahren mit einem an einem horizontalen Röhrentragarm (8) befestigten, bei Schichtaufnahmebetrieb in einer horizontalen Ebene oberhalb einer Patientenlagerungsvorrichtung (1, 2) bewegbaren Röntgenstrahler (9) und einer unterhalb der Patientenlagerungsvorrichtung (1, 2) in einer horizontalen Ebene bewegbaren Bildaufnahmeeinrichtung (3), wobei zur gegensinnigen Verschiebung von Röntgenstrahler (9) und Bildaufnahmeeinrichtung (3) eine Kupplungsvorrichtung (4, 7) vorgesehen ist, die aus zwei teleskopartig gegeneinander verschiebbaren, um eine horizontale Achse schwenkbaren Teilen (4, 7) besteht, von denen der eine (4) mit der Bildaufnahmeeinrichtung (3) und der andere (7) lösbar mit dem Röhrentragarm (8) verbunden ist, dadurch gekennzeichnet, dass der mit dem Röhrentragarm (8) lösbar verbundene Teil (7) der Kupplungsvorrichtung (4, 7) an seinem oberen Ende mit einer Querschnittsveränderung (14) versehen ist, dass am Röhrentragarm (8) eine Rastvorrichtung (15; 31; 35) befestigt ist, die in die Querschnittsveränderung eingreift, dass ein Anschlag (60) vorgesehen ist, der beim Herunterfahren des Röhrentragarms (8) den Teil (7) relativ zum Röhrentragarm (8) nach oben drückt, dass die Rastvorrichtung (15; 31; 35) und die Querschnittsveränderung (14) so ausgebildet sind, dass nur bei einer Aufwärtsbewegung des mit dem Röhrentragarm verbundenen Teils (7) der Kupplungsvorrichtung (4, 7) die Rastvorrichtung (15; 31; 35) aus der Raststellung ausrückbar ist, und dass ein Arretiermechanismus (20, 22, 25; 33; 36; 38) bei Betätigung durch den Benutzer die Rastvorrichtung (15; 31; 35) in der ausgerückten Position festhält.

2. Röntgenuntersuchungsgerät nach Anspruch 1, dadurch gekennzeichnet, dass die Querschnittsveränderung eine Nut (14) ist.

3. Röntgenuntersuchungsgerät nach Anspruch 2, dadurch gekennzeichnet, dass die Rastvorrichtung einen an einem Ende (17) angelenkten Hebel (16) umfasst, der mit einem in die Nut (14) passenden Sperrstift (15) versehen ist, dass der mit dem Röhrentragarm (8) verbindbare Teil (7) der Kupplungsvorrichtung (4, 7) zwischen dem Gelenkpunkt (17) des Hebels und dem Stift (15) geführt ist, und dass der Hebel (16) mit Federkraft nach unten gedrückt wird.

4. Röntgenuntersuchungsgerät nach Anspruch 1, dadurch gekennzeichnet, dass in dem Röhrentragarm (8) eine Hülse (30) mit sich nach oben konisch erweiternder Bohrung befestigt ist, in die das obere, sich nach oben konusförmig verbreiternde Ende des mit dem Röhrentragarm (8) verbindbaren Teils (7) der Kupplungsvorrichtung (4, 7) einführbar ist, wobei die Durchmesserzunahme bei der Hülse (30) grösser ist als bei dem Teil (7), und dass in der Hülse (30) wenigstens zwei auf dem Umfang mit Abstand zueinander sitzende, sich nach oben ebenfalls konisch erweiternde Klemmbackenteile (31) vorgesehen sind, die das obere Ende des Teils (7) aufnehmen und bei einer Abwärtsbewegung in der Hülse festhalten, wobei der Arretiermechanismus (33) die Klemmbacken in einer oberen Position festhält.

5. Röntgenuntersuchungsgerät nach einem der Ansprüche 1 bis 4, dadurch gekennzeichnet, dass im Röhrentragarm (8) eine Feder (27) angeordnet ist, die den mit dem Röhrentragarm (8) rastend verbundenen Teil (7) der Kupplungsvorrichtung (4, 7) nach unten drückt.

**Revendications**

1. Appareil d'examen à rayons X pour tomographie et autres procédés d'exposition comportant une source de rayons X fixée sur un bras de support de tube horizontal pendant la tomographie et se déplaçant au-dessus de la table d'examen dans un plan horizontal et un dispositif de prise de vues se déplaçant au-dessous de la table d'examen dans un plan horizontal, un dispositif d'accouplement étant prévu pour le déplacement en sens opposés de la source de rayons X et du dispositif de prise de vues et étant constitué par deux parties pivotant autour d'un axe horizontal pouvant être déplacées de façon télescopique, l'une par rapport à l'autre, partie dont l'une est reliée au dispositif de prise de vues et l'autre est reliée de façon amovible au bras de support de tube, caractérisé en ce que la partie du dispositif d'accouplement reliée au bras de support de tube présente, à son extrémité supérieure, une variation de section, qu'un dispositif de repos est fixé au bras de support du tube et est en prise avec la variation de section, qu'une butée est prévue et monte la partie concernant le bras de support de tube pendant la descente du bras de support de tube, que le dispositif d'arrêt et la variation des sections sont réalisés de façon que le dispositif d'arrêt ne puisse être dégagé de sa position de repos que pendant une montée de la partie du dispositif d'accouplement relié au bras de support et qu'un mécanisme d'arrêt maintient le dispositif de repos dans la position dégagée pendant l'actionnement par l'utilisateur.

2. Appareil d'examen à rayons X selon la revendication 1, caractérisé en ce que la variation de section est une rainure 14.

3. Appareil d'examen à rayons X selon la revendication 2, caractérisé en ce que le dispositif d'arrêt comprend un levier (16) fixé de façon articulée à une extrémité (17) et muni d'un arrêt (15), qui s'adapte dans la rainure (14), que la partie (7) du dispositif d'accouplement (4, 7) à relier au bras de support de tube (8) se situe entre le point d'articulation (17) du levier et de l'arrêt (15) et que le levier (16) est descendu élastiquement.

4. Appareil d'examen selon la revendication 1, caractérisé en ce que dans le bras de support de tube 8 est fixée une douille 30 présentant un alésage, qui s'élargit de la façon conique et dans lequel peut être introduite l'extrémité s'élargissant de façon conique de la partie 7 du dispositif d'accouplement (4, 7) à relier au bras de support de tube, l'accroissement de diamètre de la douille (30) étant supérieur à celui de la partie (7), et qu'au moins deux parties de mâchoires de serrage expacées (31) qui s'élargissent également de façon conique vers le haut sont prévues sur la péripherie dans la douille et absorbent l'extrémité supérieure de la partie et la maintiennent pendant une descente dans la douille, le mécanisme d'arrêtage (33) maintenant les mâchoires de serrage dans une position supérieure.

5. Dispositif d'examen à rayons X selon l'une des revendications 1 à 4, caractérisé en ce que dans le bras de support de tube (8) est prévu un ressort (27), qui fait monter la partie (7) du dispositif d'accouplement (4, 7) reliée de façon encliquetée au bras de support de tube (8).

**Claims**

1. An X-ray apparatus for producing tomographic and other radiographic images, comprising an X-ray source (9) which is connected to a horizontal tube supporting arm (8) and which is movable in a horizontal plane over a patient support device (1, 2) during tomography, and also comprising an image recording device (3) which is movable in a horizontal plane underneath the patient support device (1, 2), a coupling device (4, 7) being provided for displacing the X-ray source (9) and the image recording device (3) in opposite directions, said coupling device consisting of two parts (4, 7) which are telescopically movable with respect to one another and which are rotatable about a horizontal axis, one part (4) being connected to the image recording device (3) whilst the other part (7) is detachably connected to the tube supporting arm (8), characterized in that the part (7) of the coupling device (4, 7) which is detachably connected to the tube supporting arm (8) is provided with a cross-sectional variation (14) near its upper end, the tube supporting arm (8) having mounted thereon a latching device (15, 31, 35) which engages the cross-sectional variation, there being provided an abutment (60) which, when the tube supporting arm (8) is moved downwards, urges the part (7) upwards relative to the tube supporting (8), the latching device (15, 31, 35) and the cross-sectional variation (14) being shaped so that the latching device (15, 31, 35) can be pulled out of the latching position only in response to an upwards movement of the part (7) of the coupling device (4, 7) which is connected to the tube supporting arm (8), the latching device (15, 31, 35) being maintained in the disengaged position by a locking mechanism (20, 22, 25; 36; 38) when operated by the operator.

2. An X-ray apparatus as claimed in Claim 1, characterized in that the cross-sectional variation is formed by a groove (14).

3. An X-ray apparatus as claimed in Claim 2, characterized in that the latching device comprises a lever (16) which is pivotable near one end (17) and which carries a locking pin (15) which fits in the groove (14), the part (7) of the coupling device (4, 7) which can be connected to the tube supporting arm (8) being guided between the pivot (17) of the lever and the pin (15), the lever (16) being urged downwards by spring force.

4. An X-ray apparatus as claimed in Claim 1, characterized in that in the tube supporting arm (8) there is secured a sleeve (30) which comprises a bore which widens conically in the upward direction, and in which there can be inserted the end of the upper part (7) of the coupling device (4, 7) which is to be connected to the tube supporting arm (8) and which conically widens in the upward direction, the cross-sectional increase of the sleeve (30) being greater than that of the part (7), in the sleeve (30) there being provided at least two clamping jaws (31) which are distributed over the circumference, which also widen conically in the upward direction and which engage the upper end of the part (7) and retain it in the sleeve upon movement in the downward direction, the locing mechanism (33) retaining the clamping jaws in an upper position.

5. An X-ray apparatus as claimed in any of the Claims 1 to 4, characterized in that in the tube supporting arm (8) there is provided a spring (27) which bears downwardly on the part (7) of the coupling device (4, 7) which is connected to the tube supporting arm (8) in a latching manner.

FIG.1

FIG. 2

FIG. 3

**FIG. 5**

**FIG. 4**

FIG.6

FIG.7